# EUROPÄISCHE PATENTSCHRIFT

(11) **EP 0 884 999 B1**
(45) Veröffentlichungstag und Bekanntmachung des Hinweises auf die Patenterteilung: **04.09.2002**
(21) Anmeldenummer: 97906122.3
(22) Anmeldetag: 25.02.1997
(51) Int. Cl.: A61K 7/135

(54) **BLONDIERMITTEL**
HAIR-BLEACH AGENTS
AGENT DE DECOLORATION DES CHEVEUX

(30) Priorität: 06.03.1996 DE 19608541
(43) Veröffentlichungstag der Anmeldung: 23.12.1998
(73) Patentinhaber: Henkel Kommanditgesellschaft auf Aktien, 40589 Düsseldorf-Holthausen (DE)
(72) Erfinder: HÖFFKES, Horst, D-40595 Düsseldorf (DE); NEUHAUS, Winifried, D-40822 Mettmann (DE)
(86) Internationale Anmeldenummer: EP9700888
(87) Internationale Veröffentlichungsnummer: WO97032564

(56) Entgegenhaltungen:
- WO-A-97/07776

## Beschreibung

Die Erfindung betrifft ein Blondiermittel für menschliche Haare, das in Pulverform vorliegt.

Die Veränderung von Form und Farbe der Haare stellt einen wichtigen Bereich der modernen Kosmetik dar. Dadurch kann das Erscheinungsbild der Haare sowohl aktuellen Modeströmungen als auch den individuellen Wünschen der einzelnen Person angepaßt werden. Dabei können Dauerwell- und andere die Haarform verändernde Verfahren nahezu unabhängig vom Typ der zu behandelnden Haare eingesetzt werden. Dagegen sind Färbe- und insbesondere Blondierverfahren auf bestimmte Ausgangshaarfarben begrenzt. So eignen sich für aufhellende Verfahren, die sogenannten Blondierverfahren, im wesentlichen nur dunkelblonde oder hellere Haare. Die Grundlagen der Blondierverfahren sind dem Fachmann bekannt und können in einschlägigen Monographien, z.B. von Kh. Schrader, Grundlagen und Rezepturen der Kosmetika, 2. Auflage, 1989, Dr. Alfred Hüthig Verlag, Heidelberg, oder W. Umbach (Hrg.), Kosmetik, 2. Auflage, 1995, Georg Thieme Verlag, Stuttgart, New York, nachgelesen werden.

So werden üblicherweise feste oder pastenformige Zubereitungen mit festen Oxidationsmitteln unmittelbar vor der Anwendung mit einer verdünnten Wasserstoffperoxidlösung vermischt. Diese Mischung wird dann auf das Haar aufgebracht und nach einer bestimmten Einwirkzeit wieder ausgespült.

Die genannten Zubereitungen, die vor der Anwendung mit einer Wasserstoffperoxidlösung vermischt werden, werden im weiteren als "Blondiermittel" bzw. "Blondierpulver" bezeichnet. Alle aufgeführten Mengenangaben beziehen sich, soweit nicht anders ausgeführt, ausschließlich auf diese Zubereitungen.

Weder die pastenförmigen noch die pulverförmigen Blondiermittel, die heute auf dem Markt sind, können als optimal angesehen werden. Während die Blondierwirkung auf dem Haar als befriedigend bezeichnet werden kann, bestehen doch noch eine Reihe von Nachteilen und Problemen sowohl bei Herstellung als auch bei der Handhabung dieser Mittel. Bei pastenförmigen Mitteln, die aus Stabilitätsgründen hochviskos eingestellt werden, können insbesondere die Dosierung und das Mischungsverhalten in der Wasserstoffperoxidlösung noch nicht befriedigen. Bei pulverförmigen Mitteln stehen das Staubverhalten, sowohl bei der Konfektionierung als auch bei der Anwendung, sowie ebenfalls das Mischungsverhalten bei der Anwendung im Mittelpunkt der Verbesserungsbemühungen.

In der EP-B1-0 560 088 wurde beispielsweise vorgeschlagen, das Staubverhalten von Blondierpulvern durch Zugabe von Ölen oder flüssigen Wachsen zu verbessern. In der älteren deutschen Anmeldung 196 00 216.8 war weiterhin vorgeschlagen worden, zur Entstaubung spezielle Ether in Mengen von 4 - 20 Gew.-%, bezogen auf das gesamte Blondierpulver, einzusetzen. Dabei ging der Fachmann davon aus, daß eine optimale Einstellung des Staubverhaltens in der Regel dann erreicht wird, wenn das Entstaubungmittel in Mengen von ca. 10 Gew.-%, d.h. etwa im Bereich 7 bis 15 Gew.-%, bezogen auf das gesamte Blondierpulver, zugesetzt wurde.

Es wurde nun überraschenderweise gefunden, daß auch dann Blondierpulver mit hervorragendem Staubverhalten, sehr guter Lagerfähigkeit und weiteren vorteilhaften Eigenschaften erhalten werden, wenn diese weitaus geringere Mengen der Ether gemäß deutscher Anmeldung 196 00 216.8 enthalten.

Gegenstand der vorliegenden Erfindung sind daher Mittel zum Blondieren menschlicher Haare auf Basis mindestens einer festen Peroxoverbindung und mindestens eines festen Alkaliträgers, dadurch gekennzeichnet, daß sie als Pulver vorliegen und 0,1 - 3,9 Gew.-% eines Ethers der allgemeinen Formel (I),

X - O - Y (I)

in der X und Y unabhängig voneinander für lineare und verzweigte, gesättigte und ein- oder mehrfach ungesättigte Alkylgruppen mit jeweils 4 bis 18 Kohlenstoffatomen oder Cycloalkylgruppen, Alkylcycloalkylgruppen, Alkoxycycloalkylgruppen oder Cycloalkyl-alkylgruppen mit jeweils 8 bis 22 Kohlenstoffatomen stehen mit der Maßgabe, daß der Schmelzpunkt des Ethers unterhalb von 60 °C liegt, enthalten.

Die erfindungsgemäßen Blondiermittel enthalten als erste zwingende Komponente eine feste Peroxoverbindung. Die Auswahl dieser Peroxoverbindung unterliegt prinzipiell keinen Beschränkungen; übliche, dem Fachmann bekannte Peroxoverbindungen sind beispielsweise Ammoniumperoxidisulfat, Kaliumperoxidisulfat, Natriumperoxidisulfat, Ammoniumpersulfat, Kaliumpersulfat, Natriumpersulfat, Kaliumperoxidiphosphat, Percarbonate wie Magnesiumpercarbonat, Peroxide wie Bariumperoxid sowie Perborate, Harnstoffperoxid und Melaminperoxid. Unter diesen Peroxoverbindungen, die auch in Kombination eingesetzt werden können, sind erfindungsgemäß die anorganischen Verbindungen bevorzugt. Besonders bevorzugt sind die Peroxidisulfate, insbesondere Kombinationen aus mindestens zwei Peroxidisulfaten.

Die Peroxoverbindungen sind in den erfindungsgemäßen Blondiermitteln bevorzugt in Mengen von 20-80 Gew.-%, insbesondere in Mengen von 40-70 Gew.-%, enthalten.

Als weitere zwingende Komponente enthalten die erfindungsgemäßen Blondiermittel ein Alkalisierungsmittel, das zur Einstellung des alkalischen pH-Wertes der Anwendungsmischung dient. Erfindungsgemäß können die dem Fachmann ebenfalls fiir Blondiermittel bekannten, üblichen Alkalisierungsmittel wie Ammonium-, Alkalimetall- und Erdalkalimetallhydroxyde, -carbonate, -hydrogencarbonate, - hydroxy- carbonate, -silikate, insbesondere -metasilikate, sowie Alkaliphosphate verwendet werden. In einer bevorzugten Ausführungsform enthalten die erfindungsgemäßen Blondierpulver mindestens zwei unterschiedliche Alkalisierungsmittel. Dabei können Mischungen beispielsweise aus einem Metasilikat und einem Hydroxycarbonat bevorzugt sein.

Die erfindungsgemäßen Blondiermittel enthalten Alkalisierungsmittel bevorzugt in Mengen von 10-30 Gew.-%, insbesondere 15-25 Gew.-%.

Erfindungswesentlich ist die dritte zwingende Komponente der Blondierpulver, der Ether der Formel (I). Als erfindungsgemäß besonders geeignet innerhalb dieser Gruppe haben sich solche Ether erwiesen, bei denen X und Y unabhängig voneinander für lineare und verzweigte, gesättigte und ein- oder mehrfach ungesättigte Alkylgruppen mit jeweils 4 bis 18 Kohlenstoffatomen stehen. Solche Ether sind beispielsweise Di-n-octylether und Di-n-dodecylether. Besonders bevorzugt sind solche Ether, bei denen X und Y unabhängig voneinander für lineare gesättigte Alkylgruppen mit 6 bis 14 Kohlenstoffatomen stehen. Ein erfindungsgemäß ganz besonders bevorzugter Ether ist der Di-n-octylether, der unter der Bezeichnung Cetiol® OE kommerziell erhältlich ist.

Die erfindungsgemäßen Blondiermittel enthalten die Ether der Formel (I) bevorzugt in einer Menge von 0,5 - 2,4, insbesondere 0,5 - 1,5 Gew.-%, bezogen auf das gesamte Blondiermittel.

Weiterhin hat es sich als vorteilhaft erwiesen, wenn die erfindungsgemäßen Blondiermittel nichtionogene grenzflächenaktive Stoffe enthalten. Dabei sind solche grenzflächenaktive Stoffe, die einen HLB-Wert von 5,0 und größer aufweisen, bevorzugt. Für die Definition des HLB-Wertes wird ausdrücklich auf die Ausführungen in Hugo Janistyn, Handbuch der Kosmetika und Riechstoffe, III. Band: Die Körperpflegemittel, 2. Auflage, Dr. Alfred Hüthig Verlag Heidelberg, 1973, Seiten 68-78 und Hugo Janistyn, Taschenbuch der modernen Parfümerie und Kosmetik, 4. Auflage, Wissenschaftliche Verlagsgesellschaft m.b.H. Stuttgart, 1974, Seiten 466-474, sowie die darin zitierten Originalarbeiten Bezug genommen.

Besonders bervorzugte nichtionogene oberflächenaktive Substanzen sind dabei wegen der einfachen Verarbeitbarkeit Substanzen, die kommerziell als Feststoffe oder Flüssigkeiten in reiner Form erhältlich sind. Die Definition für Reinheit bezieht sich in diesem Zusammenhang nicht auf chemisch reine Verbindungen. Vielmehr können, insbesondere wenn es sich um Produkte auf natürlicher Basis handelt, Mischungen verschiedener Homologen eingesetzt werden, beispielsweise mit verschiedenen Alkylkettenlängen, wie sie bei Produkten auf Basis natürlicher Fette und Öle erhalten werden. Auch bei alkoxylierten Produkten liegen üblicherweise Mischungen unterschiedlicher Alkoxylierungsgrade vor. Der Begriff Reinheit bezieht sich in diesem Zusammenhang vielmehr auf die Tatsache, daß die gewählten Substanzen bevorzugt frei von Lösungsmitteln, Stellmitteln und anderen Begleitstoffen sein sollen.

Bevorzugte nichtionogene grenzflächenaktive Stoffe sind
- alkoxylierte Fettalkohole mit 8 bis 22, insbesondere 10 bis 16, Kohlenstoffatomen in der Fettalkylgruppe und 1 bis 30, insbesondere 1 bis 15, Ethylenoxidund/oder Propylenoxid-Einheiten. Bevorzugte Fettalkylgruppen sind beispielsweise Lauryl-, Myristyl-, Cetyl-, aber auch Stearyl-, Isostearyl- und Oleylgruppen. Besonders bevorzugte Verbindungen dieser Klasse sind beispielsweise Laurylalkohol mit 2 bis 4 Ethylenoxid-Einheiten, Oleyl- und Cetylalkohol mit jeweils 5 bis 10 Ethylenoxideinheiten, Cetyl- und Stearylalkohol sowie deren Mischungen mit 10 bis 30 Ethylenoxideinheiten sowie das Handelsprodukt Aethoxal®B (Henkel), ein Laurylalkohol mit jeweils 5 Ethylenoxid- und Propylenoxideinheiten. Neben den üblichen alkoxylierten Fettalkoholen können auch sogenannte "endgruppenverschlossene" Verbindungen erfindungsgemäß eingesetzt werden. Bei diesen Verbindungen weist die Alkoxygruppe am Ende keine OH-Gruppe auf, sondern ist in Form eines Ethers, insbesondere eines C1-C4-Alkyl-Ethers, "verschlossen". Ein Beispiel für eine solche Verbindung ist das Handelsprodukt Dehypon®LT 054, ein C₁₂₋₁₈-Fettalkoholol + 4,5 Ethylenoxid-butylether.
- alkoxylierte Fettsäuren mit 8 bis 22, insbesondere 10 bis 16, Kohlenstoffatomen in der Fettsäuregruppe und 1 bis 30, insbesondere 1 bis 15, Ethylenoxid- und/oder Propylenoxid-Einheiten. Bevorzugte Fettsäuren sind beispielsweise Laurin-, Myristin-, Palmitin-, Stearin-, Isostearin- und Ölsäure.
- alkoxylierte, bevorzugt propoxylierte und insbesondere ethoxylierte, Mono-, Diund Triglyceride. Beispiele für bevorzugte Verbindungen sind Glycerinmonolaurat + 20 Ethylenoxid und Glycerinmonostearat + 20 Ethylenoxid.
- Polyglycerinester und alkoxylierte Polyglycerinester. Bevorzugte Verbindungen dieser Klasse sind beispielsweise Poly(3)glycerindiisostearat
   (Handelsprodukt: Lameform®TGI (Henkel)) und Poly(2)glycerinpolyhydroxystearat (Handelsprodukt: Dehymuls®PGPH (Henkel)).
- Sorbitan-Fettsäureester und alkoxylierte Sorbitan-Fettsäureester wie beispielsweise Sorbitanmonolaurat und Sorbitanmonolaurat + 20 Ethylenoxid (EO).
- Alkylphenole und Alkylphenolalkoxylate mit 6 bis 21, insbesondere 6 bis 15, Kohlenstoffatomen in der Alkylkette und 0 bis 30 Ethylenoxid- und/oder Propylenoxid-Einheiten. Bevorzugte Vertreter dieser Klasse sind beipielsweise Nonylphenol + 4 EO, Nonylphenol + 9 EO, Octylphenol + 3 EO und Octylphenol + 8 EO.

Besonders bevorzugte Klassen an nichtionogenen grenzflächenaktiven Stoffen stellen die alkoxylierten Fettalkohole, die alkoxylierten Fettsäuren sowie die Alkylphenole und Alkylphenolalkoxylate dar.

Als besonders vorteilhaft haben sich erfmdungsgemäße Mittel erwiesen, die nichtionogene grenzflächenaktive Substanzen in Mengen von 0,5 - 10 Gew.-% enthalten.

Weiterhin können die erfindungsgemäßen Blondiermittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, kationischen oder nichtionischen Tensiden auszuwählen. Anionische Tenside können dabei ganz besonders bevorzugt sein.

Bevorzugte anionische Tenside sind Alkylsulfate, Ethercarbonsäuresalze mit 10 bis 18 C-Atomen in der Alkylgruppe und bis zu 12 Glykolethergruppen im Molekül wie C₁₂H₂₅-(C₂H₄O)₆-CH₂-COONa sowie insbesondere Salze von gesättigten und speziell ungesättigten C8-C22-Carbonsäuren wie Ölsäure, Stearinsäure, Isostearinsäure und Palmitinsäure.

Diese anionischen Tenside sollten bevorzugt in fester, insbesondere Pulverform vorliegen. Ganz besonders bevorzugt sind dabei bei Raumtemperatur feste Seifen, insbesondere Natriumstearat. Diese liegen bevorzugt in Mengen von 5 bis 20 Gew.-%, insbesondere 10 bis 15 Gew.-.%, vor.

Als nichtionische Tenside eignen sich insbesondere C8-C22-Alkylmono- und oligoglycoside und deren ethoxylierte Analoga. Insbesondere die nichtethoxylierten Verbindungen, die zudem in Pulverform kommerziell erhältlich sind, haben sich als besonders geeignet erwiesen.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie das unter der Bezeichnung Dehyquart®F 75 in Abmischung mit Cetearylalkohle erhältliche Distearoylethylhydroxyethylammoniummethosulfat.

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

### Weitere Wirk-, Hilfs- und Zusatzstoffe sind beispielsweise

- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether und andere, als Feststoff stabile bzw. im Handel erhältliche Verbindungen,
- zwitterionische und amphotere Polymere, die als Feststoffe stabil bzw. bevorzugt als Handelsprodukte erhältlich sind,
- anionische Polymere wie beispielsweise Polyacrylsäuren, vernetzte Polyacrylsäuren und Vinylacetat/Crotonsäure-Copolymere, sofern diese als Feststoffe stabil bzw. bevorzugt im Handel erhältlich sind,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose, Maleinsäure und Milchsäure,
- haarkonditionierende Verbindungen wie Phospholipide, beispielsweise Sojalecithin, Ei-Lecitin und Kephaline, sowie Silikonöle
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Farbstoffe zum Einfärben der Zubereitungen,
- Wirkstoffe wie Panthenol, Pantothensäure, Allantoin, Pyrrolidoncarbonsäuren und deren Salze,
- Cholesterin,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Carbonate, Hydrogencarbonate, Guanidine, Harnstoffe sowie primäre, sekundäre und tertiäre Phosphate,

Die Auswahl dieser weiteren Stoffe wird der Fachmann gemäß der gewünschten Eigenschaften der Mittel treffen.

In einer bevorzugten Ausführungsform, insbesondere wenn das Haar nicht übermäßig beschwert werden soll, sind die erfindungsgemäßen Mittel im übrigen frei von Ölen und flüssigen Wachsen. Dabei ist klarzustellen, daß der Begriff Öle die bekannten fetten und synthetischen Öle, nicht aber Parfümöle umfaßt, die selbstverständlich in geringen Mengen als Duftstoffe eingesetzt werden können.

Die Herstellung der erfindungsgemäßen Blondiermittel kann nach den üblichen, dem Fachmann bekannten Verfahren erfolgen.

Ein bevorzugtes Verfahren besteht darin, die anorganischen, als Feststoff vorliegenden Komponenten, gegebenenfalls nach Mischung z.B. in einem Drais-Mischer, vorzulegen und mit dem grenzflächenaktiven Mittel zu besprühen. Dies erfolgt bevorzugt bei Raumtemperatur, d. h. bei Temperaturen unterhalb von ca. 30 °C; lediglich wenn die gewählten staubbindenden Komponenten bei diesen Temperaturen nicht als Flüssigkeit vorliegen, wird man erhöhte Temperaturen anwenden.

Ein weiteres Herstellungsverfahren für die erfindungsgemäßen Blondiermittel ist das Mischen aller Komponenten und die anschließende Behandlung, bevorzugt bei erhöhten Temperaturen, im Wirbelbett.

Schließlich ist es auch möglich, die erfindungsgemäßen Blondiermittel durch Vermahlen aller Komponenten in einer Kugelmühle, einer Ringwalzenmühle oder insbesondere einer Spindelmühle herzustellen.

Für die Anwendung der erfindungsgemäßen Mittel auf dem Haar werden die pulverförmigen Blondiermittel unmittelbar vor dem Auftragen mit einer Wasserstoffperoxid-Lösung vermischt. Die Konzentration dieser Wasserstoffperoxid-Lösung wird einerseits von den gesetzlichen Vorgaben und andererseits von dem gewünschten Effekt bestimmt; in der Regel werden 6- bis 12prozentige Lösungen in Wasser verwendet. Die Mengenverhältnisse von Blondierpulver und Wasserstoffperoxid-Lösung liegen dabei üblicherweise im Bereich 1:1 bis 1:2, wobei ein Überschuß an Wasserstoffperoxid-Lösung insbesondere dann gewählt wird, wenn keine zu ausgeprägte Blondierwirkung erwünscht ist.

### Beispiele

Es wurden 3 Mischungen zubereitet, deren Zusammensetzung (in g) in Tabelle 1 angegeben sind:

| Komponenten | E1 | E2 | E3 |
|---|---|---|---|
| Ammoniumperoxidisulfat | 25 | 25 | 5 |
| Kaliumperoxidisulfat | 36 | 34 | 50,5 |
| Natriummetasilikat | 18 | 18 | 18 |
| Magnesiumhydroxycarbonat | 3 | 3 | 3 |
| Natriumstearat | 11 | 11 | 11 |
| pyrogenes Siliziumdioxid | 3,5 | 3,5 | 3,5 |
| Dinatriumdihydrogenethylendiamintetaacetat | 2 | 2 | 2 |
| Di-n-octylether | 1,5 | - | 1,0 |
| Di-n-dodecylether | - | 3,5 | - |
| Laurylalkohol-3(EO)-ether | | | 6,0 |
| | | | |
| Gesamtmenge | 100 | 100 | 100 |

Alle Mittel waren absolut staubfrei und ergaben beim Vermischen mit H₂O₂-Lösungen innerhalb kurzer Zeit homogene Mischungen.

## Patentansprüche

1. Mittel zum Blondieren menschlicher Haare auf Basis mindestens einer festen Peroxoverbindung und mindestens eines festen Alkaliträgers, **dadurch gekennzeichnet, daß** es als Pulver vorliegt und 0,1 - 3,9 Gew.-% eines Ethers der allgemeinen Formel (I),
X - O - Y (I)
in der X und Y unabhängig voneinander für lineare und verzweigte, gesättigte und ein- oder mehrfach ungesättigte Alkylgruppen mit jeweils 4 bis 18 Kohlenstoffatomen oder Cycloalkylgruppen, Alkylcycloalkylgruppen, Alkoxycycloalkylgruppen oder Cycloalkyl-alkylgruppen mit jeweils 8 bis 22 Kohlenstoffatomen stehen mit der Maßgabe, daß der Schmelzpunkt des Ethers unterhalb von 60 °C liegt, enthält.

2. Mittel nach Anspruch 1, **dadurch gekennzeichnet, daß** es eine anorganische feste Peroxoverbindung enthält.

3. Mittel nach Anspruch 1 oder 2, **dadurch gekennzeichnet, daß** X und Y unabhängig voneinander für lineare und verzweigte, gesättigte und ein- oder mehrfach ungesättigte Alkylgruppen mit jeweils 4 bis 18 Kohlenstoffatomen stehen.

4. Mittel nach Anspruch 3, **dadurch gekennzeichnet, daß** X und Y unabhängig voneinander stehen fiir lineare gesättigte Alkylgruppen mit 6 bis 14 Kohlenstoffatomen.

5. Mittel nach einem der Ansprüche 1 bis 4, **dadurch gekennzeichnet, daß** der Ether der Formel (I) in einer Menge von 0,5 - 2,4 Gew.-% enthalten ist.

6. Mittel einem der Ansprüche 1 bis 5, **dadurch gekennzeichnet, daß** es zusätzlich eine nichtionogene grenzflächenaktive Substanz, ausgewählt aus
- alkoxylierten Fettalkoholen und Fettsäuren mit 8 bis 22 Kohlenstoffatomen und 1 bis 30 Ethylenoxid- und/oder Propylenoxid-Einheiten, die eine Alkylendgruppe, insbesondere eine Methylgruppe, am Ende der Alkoxygruppenkette aufweisen können,
- alkoxylierten Mono-, Di- und Triglyceriden,
- Polyglycerinestern und alkoxylierten Polyglycerinestern,
- Sorbitan-Fettsäureestern und alkoxylierten Sorbitan-Festtsäureestern,
- Alkylphenolalkoxylaten mit 6 bis 21 Kohlenstoffatomen in der Alkylkette und 0 bis 30 Ethylenoxid- und/oder Propylenoxid-Einheiten
enthält

7. Mittel nach Anspruch 6, **dadurch gekennzeichnet, daß** es die nichtionogene grenzflächenaktive Substanz in Mengen von 0,5 - 10 Gew.-% enthalten ist.

8. Mittel nach einem der Ansprüche 1 bis 7, **dadurch gekennzeichnet, daß** es bei Raumtemperatur feste Seifen, insbesondere Natriumstearat, in Mengen von 5 - 20 Gew.-%, insbesondere 10-15 Gew.-%, bezogen auf das gesamte Pulver, enthält.

9. Mittel nach einem der Ansprüche 1 bis 8, **dadurch gekennzeichnet, daß** es im übrigen frei von Ölen und flüssigen Wachsen ist, wobei Parfümöle ausgenommen sind.

## Claims

1. A preparation for blending human hair based on at least one solid peroxo compound and at least one solid alkali carrier, **characterized in that** it is a powder and contains 0.1 to 3.9% by weight of an ether corresponding to general formula (I):
X - O - Y (I)
in which X and Y independently of one another represent linear and branched, saturated and mono- or polyunsaturated alkyl groups containing 4 to 18 carbon atoms or cycloalkyl groups, alkyl cycloalkyl groups,alkoxy cycloalkyl groups or cycloalkyl alkyl groups containing 8 to 22 carbon atoms, with the proviso that the melting point of the ether is below 60°C.

2. A preparation as claimed in claim 1, **characterized in that** it contains an inorganic solid peroxo compound.

3. A preparation as claimed in claim 1 or 2, **characterized in that** X and Y independently of one another represent linear or branched, saturated and mono- or polyunsaturated alkyl groups containing 4 to 18 carbon atoms.

4. A preparation as claimed in claim 3, **characterized in that** X and Y independently of one another represent linear saturated alkyl groups containing 6 to 14 carbon atoms.

5. A preparation as claimed in any of claims 1 to 4, **characterized in that** the ether corresponding to formula (I) is present in a quantity of 0.5 to2.4% by weight.

6. A preparation as claimed in any of claims 1 to5, **characterized in that** it additionally contains a nonionic interfacially active substance selected from
- alkoxylated fatty alcohols and fatty acids containing 8 to 22 carbon atoms and 1 to 30 ethylene oxide and/or propylene oxide units which may have a terminal alkyl group, more particularly a methyl group, at the end of the alkoxy group chain,
- alkoxylated mono-, di- and triglycerides,
- polyglycerol esters and alkoxylated polyglycerol esters,
- sorbitan fatty acid esters and alkoxylated sorbitan fatty acid esters,
- alkylphenol alkoxylates containing 6 to 21 carbon atoms in the alkyl chain and 0 to 30 ethylene oxide and/or propylene oxide units.

7. A preparation as claimed in claim 6, **characterized in that** it contains the nonionic interfacially active substance in quantities of 0.5 to 10% by weight.

8. A preparation as claimed in any of claims 1 to 7, **characterized in that** it contains soaps solid at room temperature, more particularly sodium stearate, in quantities of 5 to 20% by weight and more particularly 10 to 15% by weight, based on the powder as a whole.

9. A preparation as claimed in any of claims 1 to 8, **characterized in that** it is free from oils and liquid waxes excluding perfume oils.

## Revendications

1. Agent de décoloration pour cheveux humains, à base d'au moins un composé peroxo solide et d'au moins un support alcalin solide, **caractérisé en ce qu'**il se présente sous la forme de poudre et qu'il contient 0,1 à 3,9% en poids d'un éther de la formule générale (I)
X - O - Y (I)
dans laquelle X et Y représentent indépendamment l'un de l'autre, des radicaux alcoyle linéaires et ramifiés, saturés et insaturés une ou plusieurs fois, ayant chaque fois 4 à 18 atomes de carbone ou des radicaux cycloalcoyle, des radicaux alcoylcycloalcoyle, des radicaux alcoxycycloalcoyle ou des radicaux cycloalcoylalcoyle ayant chaque fois, 8 à 22 atomes de carbone, avec la condition que le point de fusion de l'éther est inférieur à 60°C.

2. Agent suivant la revendication 1, **caractérisé en ce qu'**il contient un composé peroxo solide inorganique.

3. Agent suivant la revendication 1 ou 2, **caractérisé en ce que** X et Y représentent indépendamment l'un de l'autre, des radicaux alcoyle linéaires et ramifiés, saturés et insaturés une ou plusieurs fois, ayant chaque fois 4 à 18 atomes de carbone.

4. Agent suivant la revendication 3, **caractérisé en ce que** X et Y représentent indépendamment l'un de l'autre, un radical alcoyle linéaire saturé, ayant 6 à 14 atomes de carbone.

5. Agent suivant l'une quelconque des revendications 1 à 4, **caractérisé en ce que** l'éther de formule (I) est présent en une quantité allant de 0,5 à 2,4% en poids.

6. Agent suivant l'une quelconque des revendications 1 à 5, **caractérisé en ce qu'**il contient en outre, une substance tensioactive non ionogène, choisie parmi :
- les alcools gras et acides gras alcoxylés, ayant 8 à 22 atomes de carbone et 1 à 30 unités oxyde d'éthylène et/ou oxyde de propylène, qui peuvent présenter un radical alcoyle terminal, en particulier un radical méthyle, sur l'extrémité de la chaine du radical alcoxy ;
- les mono-, di- et triglycérides alcoxylés ;
- les esters de polyglycérine et les esters de polyglycérine alcoxylés ;
- les esters d'acide gras du sorbitan et les esters d'acide gras du sorbitan alcoxylés ;
- les alcoxylates d'alcoylphénol ayant 6 à 21 atomes de carbone dans la chaine alcoyle et 0 à 30 unités oxyde d'éthylène et/ou oxyde de propylène.

7. Agent suivant la revendication 6, **caractérisé en ce qu'**il contient la substance tensioactive non ionogène en une quantité allant de 0,5 à 10% en poids.

8. Agent suivant l'une quelconque des revendications 1 à 7, **caractérisé en ce qu'**il contient des savons solides à la température ambiante, en particulier le stéarate de sodium, en des quantités allant de 5 à 20% en poids, en particulier de 10 à 15% en poids, sur base de la poudre totale.

9. Agent suivant l'une quelconque des revendications 1 à 8, **caractérisé en ce qu'**il est du reste, exempt d'huiles et de cires liquides, où les huiles de parfum sont exceptées.
